# FASCICULE DE BREVET EUROPEEN

(11) **EP 2 083 814 B1**
(45) Date de publication et mention de la délivrance du brevet: **20.03.2013**
(21) Numéro de dépôt: 07822084.5
(22) Date de dépôt: 31.10.2007
(51) Int. Cl.: A61K 31/133, A61P 25/00

(54) **LE DMAE EN TANT QU'AGENT UNIQUE POUR LE TRAITEMENT DE DEFICIT COGNITIF LEGER**
DMAE ALS ALLEINIGES MITTEL ZUR BEHANDLUNG VON LEICHTER KOGNITIVER BEEINTRÄCHTIGUNG
DMAE AS SOLE AGENT FOR THE TREATMENT OF MILD COGNITIVE IMPAIRMENT

(30) Priorité: 31.10.2006 FR 0609548
(43) Date de publication de la demande: 05.08.2009
(73) Titulaire: Pierre Fabre Medicament, 92100 Boulogne-Billancourt (FR)
(72) Inventeur: FABRE, Pierre, 81100 Castres (FR); PRZYBYLSKI, Christophe, 31290 Villefranche De Lauragais (FR); DUBOIS, Bruno, 75016 Paris (FR)
(74) Mandataire: Regimbeau
(86) Numéro de dépôt international: PCT/EP2007/061737
(87) Numéro de publication internationale: WO 2008/053011

(56) Documents cités:
- WO-A-03/003981
- US-A1- 2006 211 721
- MARSH G R ET AL: "THE EFFECTS OF DEANOL ON COGNITIVE PERFORMANCE AND ELECTROPHYSIOLOGY IN ELDERLY HUMANS" PSYCHOPHARMACOLOGY, SPRINGER VERLAG, BERLIN, DE, vol. 66, no. 1, 1979, pages 99-104, XP009084427 ISSN: 0033-3158
- CAFFARRA ET AL.: "the effect of deanol on amnesic disorders" ACTA BIO-MED, vol. 51, 1980, pages 383-389, XP009084425
- DIMPFEL W ET AL: "SOURCE DENSITY ANALYSIS OF FUNCTIONAL TOPOGRAPHICAL EEG: MONITORING OF COGNITIVE DRUG ACTION" EUROPEAN JOURNAL OF MEDICAL RESEARCH, MUNICH, DE, vol. 1, 1996, pages 283-290, XP009084426 ISSN: 0949-2321
- VORONINA T A ET AL: "EFFECT OF NICERGOLINE ON LEARNING AND MEMORY" METHODS AND FINDINGS IN EXPERIMENTAL AND CLINICAL PHARMACOLOGY, PROUS, BARCELONA, ES, vol. 10, no. 7, juillet 1988 (1988-07), pages 431-435, XP009084428 ISSN: 0379-0355
- ALLAIN H ET AL: "Mild cognitive impairment: a treatment at last?" LANCET NEUROLOGY, LANCET PUBLISHING GROUP, LONDON, GB, vol. 3, no. 11, novembre 2004 (2004-11), page 643, XP004810174 ISSN: 1474-4422

## Description

La présente invention concerne l'utilisation à titre de principe actif unique de diméthylaminoéthanol (déanol ou DMAE) sous sa forme libre, sa forme de sels ou d'esters ainsi que leurs hydrates et solvates, pour la fabrication d'un médicament destiné au traitement d'un déficit cognitif léger non démentiel caractérisé par un syndrome amnésique de type hippocampique.

Les articles de Marsh et al. (Psychopharmacology 1979, 66(1), 99-104) et Caffarra et al. (Acta Biomed. 1980, 51, 383-389) ont montré que l'utilisation de DMAE n'avait aucun effet sur des personnes âgées normales, éventuellement atteintes de troubles cognitifs légers mais non caractérisés par un syndrome amnésique de type hippocampique, et sur des personnes atteintes de troubles amnésiques suite à un problème cérébrovasculaire ou un traumatisme crânien, respectivement.

Il est donc important de bien préciser que l'utilisation objet de la présente invention vise un médicament destiné à des sujets, et en particulier des sujets âgés, ayant une plainte cognitive (typiquement mnésique), c'est-à-dire présentant une baisse de performances aux tests cognitifs (et notamment ceux de la mémoire), et en particulier avec épargne du fonctionnement cognitif et intellectuel global et intégrité des activités de la vie de tous les jours. Ces sujets sont en outre indemnes de tous syndromes démentiels patents, ne répondant pas en particulier aux critères cliniques de la démence tel que définis dans le DSM-IV (Diagnostic and Statistical Manual of Mental Disorders, 4ème Edition), la CIM-10 (Classification internationale des maladies 10ème édition) ou Maladie d'Alzheimer possible ou probable selon le NINCDS-ADRDA (National Institute of Neurologic and Communicative Disorders and Stroke (NINCDS) and Alzheimer's Disease and Related Disorders Association (ADRDA) criteria : McKhann G, Drachman D, Folstein M, Katzman R, Price D, Stadlan EM. Clinical diagnosis of Alzheimer's disease: report of the NINCDS-ADRDA work group under the auspices of Department of Health and Human Services Task Force on Alzheimer's disease. Neurology 1984; 34: 939-44). Ces sujets présentent en outre un syndrome amnésique de type hippocampique qui peut être objectivé par une altération des résultats du test de Grober et Buschke.

La présente invention vise donc plus particulièrement l'utilisation de DMAE sous les différentes formes précitées, c'est-à-dire notamment sous la forme de sels ou d'esters choisis parmi le pyroglutamate, le chlorhydrate, l'ascorbate, le bitartrate, le dihydrogéno-phosphate, l'orotate, le succinate, le carbamate, le phénylacétate, le benzoate, le para-acétamido benzoate et l'acéglutamate.

Depuis la moitié du siècle dernier, plusieurs concepts ont été proposés pour tenter d'isoler des indicateurs de diagnostics optimaux qui permettront de discriminer au mieux les sujets âgés avec des troubles cognitifs légers et stables, de ceux évoluant vers une démence puisque tous les sujets âgés avec de légers troubles cognitifs n'évoluent pas obligatoirement vers une démence. Le but de ces concepts est d'identifier les personnes susceptibles de bénéficier d'une prise en charge au minimum préventive sinon palliative ou curative. Ces concepts basés sur différents types d'approches (cliniques, neuropsychologiques) et utilisant différents outils diagnostics (tests neuropsychologiques) ont en commun la présence d'un trouble cognitif chez le sujet âgé.

Compte tenu des résultats obtenus et du mécanisme d'action avancé, un certain bénéfice pourrait également être attendu chez des patients répondant à ces diverses classifications. A titre d'illustration, on mentionnera ci-après une liste de principales dénominations décrites dans la littérature pour les troubles cognitifs du sujet âgé.
■ Prodromal AD
■ Amnesic MCI (single domain ou multiple domain),
■ Mild Cognitive Impairment (MCI) - Petersen et al. (1999),
■ Cognitively Impaired Non Demented (CIND) - 1995,
■ Aging-Associated Cognitive Decline (AACD) - Levy (1994),
■ Mild Cognitive Decline (MCD) - OMS (1994),
■ Age-Related Cognitive Decline (ARCD) - DSM IV
■ Late-Life Forgetfulness (LLF) De Blakford et La Rue,
■ Age-Consistent Memory Impairment (ACMI),
■ Age-Associated Memory Impairment (AAMI) - Crook et al. (1986),
■ Malignant Senescent Forgetfulness (MSF),
■ Benign Senescent Forgetfulness (BSF).

L'une des dénominations les plus récentes, le Mild Cognitive Impairment (MCI) permet de caractériser un état ne correspondant plus à la normalité (déclin cognitif lié à l'âge qui serait "normal") mais pas encore à une démence (type maladie d'Alzheimer). Cliniquement, le MCI s'applique à une population de sujets âgés présentant une plainte cognitive, avec théoriquement un risque élevé d'évoluer vers une démence.

Petersen RC, "Mild Cognitive Impairment as a diagnostic entity", J Intern Med 2004 : 256 : 183-194, a proposé plusieurs définitions du trouble :
■ Plainte cognitive, corroborée par l'entourage,
■ Anormal pour l'âge,
■ Absence de démence,
■ Déclin cognitif,
■ Essentiellement activités fonctionnelles normales.

Il y a actuellement un consensus sur la classification (classification syndromique) de MCI en 4 sous-catégories :
■ MCI simple domaine amnésique (détérioration uniquement dans le domaine de la mémoire ;
■ MCI multi-domaines amnésique (détérioration dans plusieurs domaines cognitifs dont celui de la mémoire ;
■ MCI simple domaine non amnésique (détérioration dans un seul domaine cognitif autre que celui de la mémoire ;
■ MCI multi-domaines non amnésique (détérioration dans plusieurs domaines cognitifs mais épargnant celui de la mémoire).

D'un point de vue évolutif, le MCI type amnésique "simple" (single domain) et le MCI multi-domaines amnésique évolueraient principalement vers une démence Alzheimer.

D'autres définitions ont été proposées qui tendent à individualiser et caractériser le MCI par son mode évolutif. Le "MCI de type Alzheimer" ou "Maladie d'Alzheimer présymptomatique" se caractérise par :
■ une plainte mnésique du sujet ou de ses proches ;
■ un début progressif ; pas ou peu de retentissement sur les activités de la vie quotidienne (iADL) ;
■ un syndrome amnésique de type hippocampique (mauvais rappel libre malgré un bon encodage, rappel différé altéré ne bénéficiant pas de l'indiçage, multiples intrusions) ;
■ une persistance des troubles mnésiques lors d'une évaluation ultérieure ; absence de démence ;
■ et l'absence de toute autre cause potentielle de MCI (si besoin, réalisation d'examens paramédicaux : imagerie, biologie...).

La majeure partie des traitements ayant montré une certaine efficacité dans la maladie d'Alzheimer a également été testée dans le MCI, y compris les produits ayant obtenu, dans l'Autorisation de Mise sur le Marché, une indication pour le traitement de la maladie d'Alzheimer tel que les anticholinestérasiques ou la mémantine). Il est important de noter qu'à ce jour aucun de ces produits testés n'a montré d'effet spécifique dans le traitement du MCI.

On indiquera ci-après la liste des principaux médicaments pour lesquels on a reconnu une activité dans le traitement de la maladie d'Alzheimer et dont l'efficacité a également été testée dans le MCI :
■ Rofecoxib (Merck) : 3, then 4 yr, N = 1457 conversion to AD, ADAS cog, SRT, CDR, Blessed ;
■ Donepezil and Vit E (ADCS) : 3 yr, N = 769 conversion to AD ;
■ Donepezil (Pfizer) : 6 mo, N = 269 symptom change ;
■ Rivastigmine (Novartis) : 3, then 4 yr, N = 1018 conversion to AD, composite neurocognitive change ;
■ Piracetam (UCB) : 1 yr N ≈ 600 symptom progression, rate of decline ;
■ Galantamine (INT-11) (Janssen) : 2 yr, N ≈ 900 symptom progression M-ADAS cog, DSST, CDR ;
■ Galantamine (INT-18) (Janssen) : 2 yr, N ≈ 1000 symptom progression M-ADAS cog, DSST, CDR and MRI ;
■ CX 516, AMPA modulator (Cortex, Servier) : 1 mo., N = 168 Symptomatic.

Aucune expérimentation conduite avec ces médicaments sur le MCI n'a démontré d'effet significatif sur les principaux critères, ni sur les délais de conversion. La plupart des essais ayant été réalisés avec comme critère principal, le taux de conversion (Schneider, Mild Cognitive Impairment, Am. J. Geriatr. Psychiatry. 2005 ; 13: 629-632).

C'est ainsi que la Demanderesse a constaté de façon surprenante que le déficit cognitif léger non démentiel, en particulier le MCI, se caractérisant essentiellement par un trouble isolé de la mémoire, était un modèle spécifique bénéficiant de façon optimale des effets thérapeutiques du DMAE et de ses sels et autres dérivés tels qu'esters, ainsi que de ses hydrates et solvates, en démontrant un effet spécifique sur l'amélioration de la mémoire.

La population cible est celle des sujets présentant un déficit cognitif léger avec un syndrome amnésique de type hippocampique objectivé par une altération des résultats du test de Grober et Buschke appelé encore test de rappel libre/rappel indicé à 16 items (RL/RI-16 items) (Van der Linden M. et al., L'évaluation des troubles de la mémoire, Eds Solal, p 25-45). Ainsi, ce test permet de sélectionner la population cible.

En effet, le syndrome amnésique de type hippocampique est défini par les caractéristiques suivantes (Dubois B. et al., Lancet Neurol., 2004, 246-248; Dubois B. et al., Lancet Neurol., 2007, 734-746) :
- déficit du rappel libre malgré un encodage contrôlé,
- inefficacité de l'indiçage ou atteinte de la reconnaissance témoignant d'un défaut de stockage,
- nombreuses intrusions.

Le test de Grober et Buschke permet de contrôler les étapes d'encodage et de récupération afin d'isoler une étape de stockage défaillante, ce qui en fait un outil très performant pour authentifier un syndrome amnésique de type hippocampique.

Ce test se déroule de la manière suivante :

Une liste de 16 mots (ou items), appartenant à 16 catégories sémantiques différentes, est à mémoriser selon 4 phases :
1. une phase de présentation des 16 mots, présentés 4 par 4, correspondant à une phase d'identification et d'encodage contrôlé des 16 mots avec rappel immédiat, suivie d'une tâche distractrice (20s, par exemple, comptage à rebours par 1, à partir de 374),
2. une phase de trois rappels libres et de trois rappels indicés appliquée à l'ensemble des 16 mots (l'indiçage catégoriel utilisé lors de l'encodage est fourni pour les items non évoqués en rappel libre), avec une tâche distractrice de 20 secondes entre chaque rappel,
3. une phase de reconnaissance des 16 items parmi les 16 items, 16 distracteurs sémantiques et 16 distracteurs neutres, et
4. une phase de rappel libre/indicé après 20 minutes.

Phase 1 : Les mots sont présentés quatre par quatre sur une fiche soit quatre fiches. Durant la phase d'« encodage », le psychologue présente au patient une fiche à la fois. Pour chaque fiche de quatre items, le psychologue demande au patient de chercher et de lire à voix haute l'item (par exemple, jonquille) correspondant à l'indice catégoriel fourni (par exemple, fleur). Quand les quatre items d'une fiche ont été correctement identifiés, le psychologue retire la fiche et administre un test de « rappel indicé immédiat » des quatre mots : il fournit l'indice catégoriel à voix haute et le patient doit rappeler l'item appartenant à cette catégorie. Si certains mots ne sont pas récupérés lors de ce « rappel indicé immédiat », le psychologue présente de nouveau la fiche correspondante et donne le nom de la catégorie de l'item non rappelé afin que le patient identifie et lise l'item cible. Ensuite, le psychologue procède de nouveau à un « rappel indicé immédiat» en cachant la fiche et en demandant au patient de rappeler l'item, correspondant à la catégorie, non cité au premier essai. Trois essais de « rappel indicé immédiat » peuvent être proposés pour chaque item. Le psychologue procède de la même manière pour les trois autres fiches de quatre items. Après la présentation et le rappel indicé immédiat réussi de la quatrième fiche, le patient est soumis à une tâche distractrice de 20 secondes (comptage à rebours).

Phase 2: Le psychologue procède ensuite à la phase de rappel libre lors de laquelle le patient doit rappeler l'ensemble des mots qui lui ont été présentés précédemment sur les fiches, dans n'importe quel ordre. Cette phase de rappel libre dure deux minutes puis le psychologue procède à la phase de « rappel indicé » uniquement pour les mots non rappelés en phase de « rappel libre ». Le psychologue donne alors la catégorie de ces mots pour que le patient puisse rappeler l'item correspondant. Le cas échéant, le psychologue fournit la réponse correcte. La même procédure de « rappel libre » et de « rappel indicé » avant la tâche distractrice est répétée trois fois afin d'obtenir trois essais de rappel. Au troisième essai, le patient n'est pas corrigé.

Phase 3 : Le psychologue lui propose alors la phase de « reconnaissance » juste après la tâche distractrice. Le patient doit reconnaître les 16 items cibles parmi 32 distracteurs.

Phase 4 : Vingt minutes après la phase de reconnaissance, se déroule une phase de « rappel libre » et de « rappel indicé ».

Ce test a donc pour but :
● de contrôler les troubles attentionnels par la procédure de rappel indicé immédiat,
● de distinguer la capacité à récupérer des mots spontanément (rappel libre) de la capacité à stocker (rappel indicé) et à consolider les mots (rappel indicé après 20 minutes de délai).

La population cible présentant un syndrome amnésique de type hippocampique est alors sélectionnée selon les critères suivants :
- une note de rappel libre inférieure ou égale à 20, cette note étant obtenue en dénombrant les items correctement cités lors des trois essais de rappel libre de la phase 2, et
- une note de rappel total inférieure ou égale à 40, cette note étant obtenue en dénombrant les items correctement cités lors des trois essais de rappel libre et de rappel indicé de la phase 2.

Dans le cadre de la présente invention, l'activité du DMAE et de ses sels et esters, ainsi que de ses hydrates et solvates a été évaluée sur une population chez qui ce trouble mnésique a été induit par injection de scopolamine pour reproduire le profil de la population cible.

La Demanderesse a notamment réalisé une étude chez 24 sujets sains (en cross-over), sur les effets du DMAE ainsi que de certains sels et esters particuliers et notamment du pyroglutamate de DMAE (pGlu-DMAE), après administration de doses répétées, sur les troubles mnésiques induits par injection de scopolamine bloquant la transmission cholinergique. Les médicaments administrés ont été présentés sous des formes aptes à l'administration par voie orale correspondant à des unités de dosage permettant des administrations journalières de l'ordre de 100 mg à 3 g, avantageusement de 300 mg à 3 g de principe actif chez le sujet adulte et en particulier de l'ordre de 1500 mg par jour. Les médicaments testés ont conduit à un taux plasmatique correspondant à un pic Cmax d'environ 1,5 µg/ml de DMAE et/ou d'environ 24 ng/ml de pGlu (acide pyroglutamique).

La Demanderesse a réalisé les tests suivants :
■ Test de la mémoire ; Buschke Selective Reminding test,
■ Test d'attention ; Digit Symbol Substitution Test (90"),
■ Evaluation de la vigilance et de l'humeur : EVA de Bond et Lader,
■ Tests de performance sensori-motrice : temps de réaction (total, décisionnel et moteur).

Les principaux résultats observés ont été consignés sur les graphes mentionnés aux figures annexées 1 à 4 sur lesquelles :
■ LTS désigne le nombre de mots acquis en mémoire à long terme,
■ LTR désigne le nombre de mots récupérés dans la mémoire à long terme pour chaque essai, c'est-à-dire les mots appartenant à LTS et qui sont redits pour l'essai en cours, et
■ CLTR désigne le nombre de mots qui sont acquis définitivement, c'est-à-dire qui seront rappelés lors des essais suivants.

Le DMAE ainsi que ses dérivés, en particulier le pyroglutamate de DMAE, a montré un effet spécifique sur les performances de la mémoire sans effet sur les autres composants cognitifs testés, tels que la vigilance, l'attention, etc.

Par conséquent, le mécanisme d'action du DMAE ou de ses sels apparaît différent de celui des anticholinestérasiques classiques précités dont l'effet sur la mémoire n'apparaît que comme la résultante d'un effet global sur divers aspects cognitifs tels que la vigilance, l'attention, l'apprentissage, etc.

A la suite de diverses autres expérimentations menées par la Demanderesse, il apparaît que le DMAE et ses sels ou esters à activité procholinergique, semblent agir plus spécifiquement par la voie cholinergique septo-hippocampique, impliquée dans les processus mnésiques, plutôt que sur le système cholinergique innominato-cortical, plus impliqué dans le processus intentionnel compte tenu de sa distribution frontale.

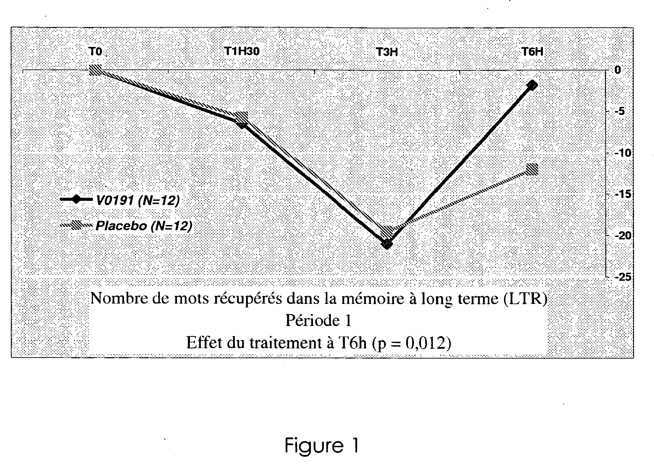

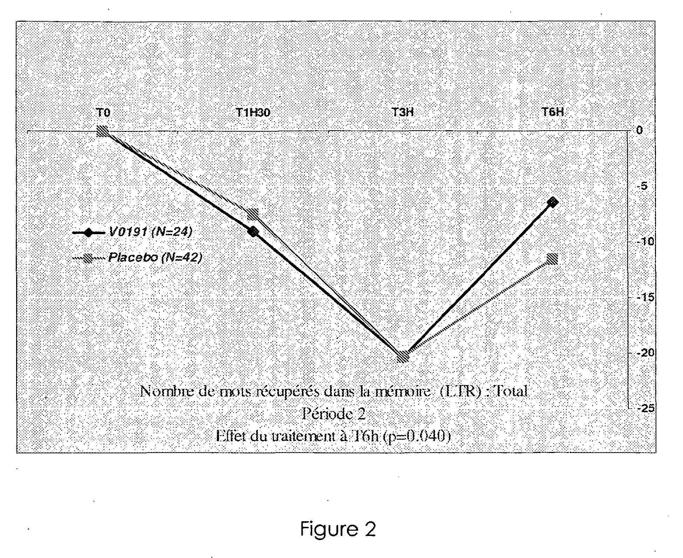

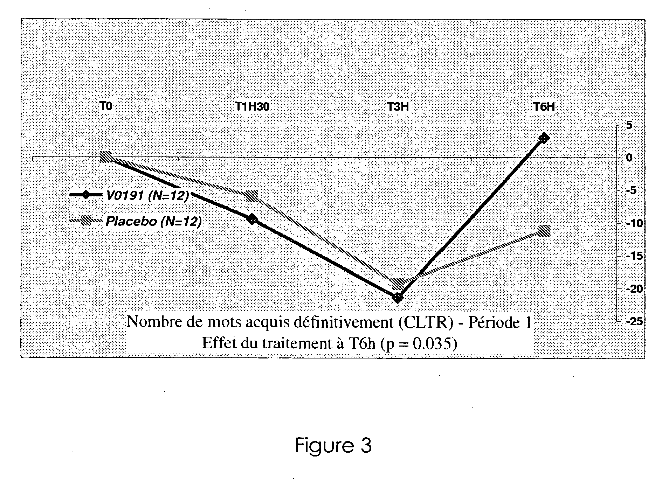

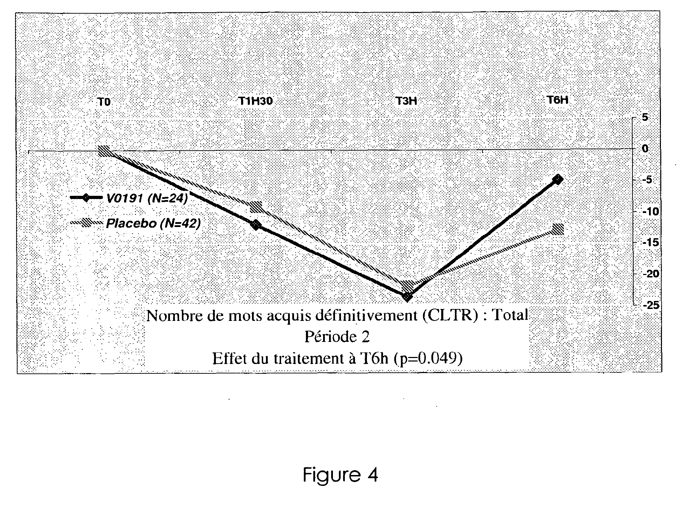

## Revendications

1. Utilisation à titre de principe actif unique de diméthylaminoéthanol (DMAE) sous forme libre, de sels ou d'esters ainsi que de leurs hydrates et solvates, pour la fabrication d'un médicament destiné au traitement d'un déficit cognitif léger non démentiel **caractérisé par** un syndrome amnésique de type hippocampique.

2. Utilisation selon la revendication 1, **caractérisée en ce que** ledit DMAE est utilisé sous la forme d'un sel ou ester choisi parmi les dérivés suivants : le pyroglutamate, le chlorhydrate, l'ascorbate, le bitartrate, le dihydrogéno phosphate, l'orotate, le succinate, le carbamate, le phénylocétate, le benzoate, le para-acétamido benzoate et l'acéglutamate.

3. Utilisation selon l'une des revendications 1 et 2, **caractérisée en ce que** le médicament est présenté sous une forme destinée à l'administration orale d'une unité de dosage permettant l'administration journalière de 300 mg à 3g, en particulier de 1500 mg, de principe actif chez un sujet adulte.

4. Utilisation selon la revendication 3, **caractérisée en ce que** le médicament est présenté sous une forme d'unité de dosage pour administration par voie orale permettant une administration journalière de diméthylaminoéthanol conduisant à un taux plasmatique correspondant à une Cmax d'environ 1,5 µg/ml de diméthylaminoéthanol.

5. Utilisation selon la revendication 2, **caractérisée en ce qu'**on utilise le pyroglutamate de déanol.

6. Utilisation selon la revendication 5, **caractérisée en ce que** le médicament est présenté sous une forme destinée à l'administration orale d'une unité de dosage permettant l'administration journalière de 300 mg à 3g, en particulier de 1500 mg de pyroglutamate de déanol chez un sujet adulte.

7. Utilisation selon la revendication 6, **caractérisée en ce que** le médicament est présenté sous une forme d'unité de dosage pour administration par voie orale permettant une administration journalière de pyroglutamate de diméthylaminoéthanol conduisant à un taux plasmatique correspondant à une Cmax d'environ 1,5 µg/ml de diméthylaminoéthanol et un taux plasmatique correspondant à un Cmax d'environ 24 ng/ml d'acide pyroglutamique.

## Claims

1. Use of dimethylaminoethanol (DMAE) as sole active ingredient in free form, in the form of its salts or esters and their hydrates and solvates, for the manufacture of a medicinal product intended for the treatment of non-dementia mild cognitive impairment, **characterized by** an amnesic syndrome of hippocampal type.

2. Use according to claim 1, **characterized in that** said DMAE is used in the form of a salt or ester chosen from among the following derivatives: pyroglutamate, hydrochloride, ascorbate, bitartrate, dihydrogen phosphate, orotate, succinate, carbamate, phenylacetate, benzoate, para-acetamido benzoate and aceglutamate.

3. Use according to any of claims 1 and 2, **characterized in that** the medicinal product is presented in a form intended to be taken via oral route, in a dosage unit allowing a daily dose for adults of 300 mg to 3 g, in particular 1,500 mg, of active ingredient.

4. Use according to claim 3, **characterized in that** the medicinal product is in the form of a dosage unit to be taken via oral route, allowing the daily administration of dimethylaminoethanol leading to a plasma level corresponding to a Cmax of around 1.5 µg/ml of dimethylaminoethanol.

5. Use according to claim 2, **characterized in that** deanol pyroglutamate is used.

6. Use according to claim 5, **characterized in that** the medicinal product is presented in a form intended to be taken by oral route in a unit dosage allowing a daily administration for adults of 300 mg to 3 g, in particular 1,500 mg of deanol pyroglutamate.

7. Use according to claim 6, **characterized in that** the medicinal product is presented in the form of a dosage unit to be taken via oral route, allowing the daily administration of dimethylaminoethanol pyroglutamate leading to a plasma level corresponding to a Cmax of around 1.5 µg/ml of dimethylaminoethanol and a plasma level corresponding to a Cmax of around 24 ng/ml of pyroglutamic acid.

## Patentansprüche

1. Verwendung von Dimethylaminoethanol (DMAE) in freier Form, von dessen Salzen oder Estern sowie deren Hydraten und Solvaten, als einziger Wirkstoff für die Herstellung eines Medikaments zur Behandlung eines nicht-dementiellen leichten kognitiven Defizits, das durch ein amnesisches Syndrom vom Hippocampus-Typ gekennzeichnet ist.

2. Verwendung nach Anspruch 1, **dadurch gekennzeichnet, dass** das DMAE in Form eines Salzes oder Esters verwendet wird, das/der aus den folgenden Derivaten ausgewählt ist: Pyroglutamat, Chlorhydrat, Ascorbat, Bitartrat, Dihydrogenphosphat, Orotat, Succinat, Carbamat, Phenylacetat, Benzoat, para-Acetamidobenzoat und Aceglutamat.

3. Verwendung nach einem der Ansprüche 1 und 2, **dadurch gekennzeichnet, dass** das Medikament in einer zur oralen Verabreichung bestimmten Form vorliegt, mit einer Dosiseinheit, welche die Verabreichung einer Tagesdosis von 300 mg bis 3 g, insbesondere von 1500 mg des Wirkstoffs bei einem erwachsenen Subjekt ermöglicht.

4. Verwendung nach Anspruch 3, **dadurch gekennzeichnet, dass** das Medikament in Form einer Dosiseinheit zur oralen Verabreichung vorliegt, welche die Verabreichung einer Tagesdosis von Dimethylaminoethanol ermöglicht, die zu einem Plasmaspiegel entsprechend einem Cmax von etwa 1,5 µg/ml Dimethylaminoethanol führt.

5. Verwendung nach Anspruch 2, **dadurch gekennzeichnet, dass** Deanolpyroglutamat verwendet wird.

6. Verwendung nach Anspruch 5, **dadurch gekennzeichnet, dass** das Medikament in einer zur oralen Verabreichung bestimmten Form vorliegt, mit einer Dosiseinheit, welche die Verabreichung einer Tagesdosis von 300 mg bis 3 g, insbesondere von 1500 mg Deanolpyroglutamat bei einem erwachsenen Subjekt ermöglicht.

7. Verwendung nach Anspruch 6, **dadurch gekennzeichnet, dass** das Medikament in Form einer Dosiseinheit zur oralen Verabreichung vorliegt, welche die Verabreichung einer Tagesdosis von Dimethylaminoethanol-Pyroglutamat ermöglicht, die zu einem Plasmaspiegel entsprechend einem Cmax von etwa 1,5 µg/ml Dimethylaminoethanol und einem Plasmaspiegel entsprechend einem Cmax von etwa 24 ng/ml Pyroglutaminsäure führt.
